(19) Europäisches Patentamt
European Patent Office
Office européen des brevets

(11) Publication number: **0 076 722**
**B1**

(12) **EUROPEAN PATENT SPECIFICATION**

(45) Date of publication of patent specification: **20.02.85**

(21) Application number: **82401703.2**

(22) Date of filing: **20.09.82**

(51) Int. Cl.⁴: **C 07 C 51/10,** C 07 C 57/30, C 07 C 57/58, C 07 C 59/64, C 07 C 51/41, B 01 J 31/28 // A61K31/195

(54) **Process for the preparation of alpha-aryl-propionic acids and their alkaline salts.**

(30) Priority: **21.09.81 IT 2405481**

(43) Date of publication of application:
**13.04.83 Bulletin 83/15**

(45) Publication of the grant of the patent:
**20.02.85 Bulletin 85/08**

(84) Designated Contracting States:
**BE CH DE FR GB LI NL**

(56) References cited:
**CH-A- 589 593**
**DE-A-2 828 041**
**DE-B-2 600 541**
**GB-A- 988 954**
**GB-A-2 079 748**
**US-A-4 102 920**
**US-A-4 102 921**

(73) Proprietor: **Montedison S.p.A.**
**Patents & Licensing Dept. Foro Buonaparte, 31**
**P.O. Box 10528**
**I-20121 Milan (IT)**

(72) Inventor: **Gardano, Andrea**
**11 C.so Roma**
**Trino Vercellese Vercelli (IT)**
Inventor: **Francalanci, Franco**
**7 C;so Torino**
**Novara (IT)**
Inventor: **Foa', Marco**
**19 via del Sabbione**
**Novara (IT)**

(74) Representative: **Hirsch, Marc-Roger**
**34 rue de Bassano**
**F-75008 Paris (FR)**

EP 0 076 722 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention concerns a process for the preparation of alpha-aryl-propionic acids and of their alkaline salts.

More particularly the present invention concerns a process for the preparation of alpha-aryl-propionic acids and their alkaline salts, by means of a process of synthesis initially using corresponding organic halides and carbon monoxide in the presence of a catalytic system based on cobalt carbonyl complexes and alkaline metal hydroxides.

The acid is easily obtained from the alkaline salt by acidification, extraction, etc., according to conventional methods.

The process is based on the carbonylation reaction of 1-halogen-1-arylethanes and involves the insertion of the carboxylic group on a secondary carbon atom, under catalytic conditions provided by Co-carbonyl complexes, and in the presence of alkaline hydroxides.

The carboxylic organic compounds, obtained according to the present invention, presents the following general formula (I):

$$\underset{\displaystyle CH_3}{\text{Ar—}\overset{}{\underset{|}{\text{CH}}}\text{—COOH}} \qquad (I)$$

wherein: Ar represents either an aromatic or a heteroaromatic group containing one or several rings linked together, having overall up to 20 carbon atoms, such as phenyl, naphthyl, diphenyl and thienyl groups.

Said Ar group may in turn be substituted by groups inert under reaction conditions. Compatible groups are, for instance: alkyl, cycloalkyl or aryl groups, each of their optionally substituted, halogen, alkoxyl, phenoxyl, ketone groups, etc.

The compounds thus obtained are worthwhile for their wide range of commercial applications.

In fact they constitute important products for carrying out organic syntheses in general, with specific possibilities in the field of so-called high-purity chemicals, phytodrugs, and especially in the field of pharmaceutical products.

More particularly, products falling within this class, such as for instance 2-(4'-isobutylphenyl)-propionic acid and 2-(6'-methoxy-2'-naphthyl)-propionic acid, are of particular interest in the field of pharmaceutical products, as antiphlogistical, analgesical, antipyrethical agents.

As previously stated, the process of the present invention is based on the carbonylation reaction conducted on a secondary benzyl halide of formula (II):

$$\underset{\displaystyle CH_3}{\text{Ar—}\underset{|}{\text{CH}}\text{—X}} \qquad (II)$$

wherein: Ar has already been defined, and X is Cl or Br, in the presence of a catalytic system consisting of Co-carbonyl complexes, further defined herein-below.

Research has only recently turned towards carbonylation of substrates of the secondary benzyl type, such as for instance secondary aryl-alkyl halides, This type of carbonylation which besides raising problems as to the reaction mechanism involved, also has the drawbacks of leading to operational difficulties as to possible yields, etc.

Prior Art literature on the subject is not particularly extensive especially with regard to commercial production.

Practically however it can be stated that in the prior art up to now the alpha-aryl-propionic acids and the alkaline salts thereof, object of the present invention, were prepared by hydrolysis of the corresponding nitriles; or by reaction of $CO_2$ with Grignard compounds; or by the decarbonylation of malonic derivatives or else by oxidation of structurally suitable alcohols or aldehydes, or finally by reduction of aryl-acrylic acids, etc.

These methods, technologically distinct from the process of the present invention, are characterized in that they are multi-step, non-catalytic methods, particularly complicated by the use of reactants not easily available and/or manipulable; they consequently have corresponding operational and economical drawbacks which make such methods of little commercial interest.

On the other hand, catalytic methods for the preparation of alpha-aryl-propionic acids have recently been suggested.

According to one of these methods, substituted alpha-aryl-propionic acids or alkyl esters thereof are prepared by means of either hydrocarbonylation or hydrocarbalkoxylation of substituted aryl-ethylenes in an aqueous or alcohol medium, catalyzed by palladium complexes and preferably in the presence of acids.

Nevertheless, the commercial interest offered by the above mentioned method does not seem high.

In fact, the method, among other difficulties, foresees the use of expensive Pd complexes as catalysts and of very high CO pressures.

In the prior art possible carbonylation reactions of halogen derivatives of the type formula (II) with Pd and Co complexes are indicated.

In the first case, the use of expensive catalysts was foreseen such as arsinic Pd complexes, under CO pressure. Moreover, the esters obtained have relatively poor yields and selectivites.

In the second case, the method foresees the formation of esters using alkaline alcoholates in the presence of dicobalt-octacarbonyl. In this case the yields and selectivities obtained are also rather poor. In fact, substantial quantities of linear acid ethers and esters are obtained. However, this involves operational drawbacks in the separation and purification of the products.

Moreover, a further economical drawback is represented by the use of alcoholates under strictly controlled pH conditions.

Further, the reaction proves to be limited to the phenyl derivatives only, possibly only alkyl-substituted derivatives.

The object of the present invention is to provide a simple and inexpensive catalytic process for large scale preparation of the acids of formula (I) or of their alkaline salts, that is free of the drawbacks cited in the discussed Prior Art.

In fact, according to the present invention, a process is provided for the carbonylation of secondary benzyl-substrates of formula (II) on the secondary carbon atom, under simple operational conditions, by using as a catalyst an inexpensive cobalt hydrocarbonyl salt that may be supported and thus easily removed, allowing it to be recycled as the catalyst in a successive cycle, and by using inexpensive and practical basic agents in an anhydrous alcohol medium. A process it thereby achieved which will ensure applicational flexibility and allows selective obtention and satisfactory yields of a much wider range of alpha-aryl-propionic acid products and/or of their alkaline salts, practically operating at a lower temperature and under atmospheric CO-pressure.

These and still other objects and advantages, which will appear more clearly to those skilled in the Art in the following description, are achieved, according to this invention, by a process for the preparation of alpha-aryl-propionic acids of formula (I) as previously defined, by means of catalytic reaction of a halide of the secondary benzyl type of formula (II), as previously defined, with carbon monoxide, characterized in that the reaction is conducted in an anhydrous alcohol solvent medium consisting of alkanols having up to 4 carbon atoms in the presence of alkaline hydroxides, and in the presence of a catalytic system consisting of a cobalt hydrocarbonyl salt or of one of its precursors, at a temperature comprised between 0°C and about 50°C, under a substantially atmospheric pressure.

The catalytic system consisting of a cobalt hydrocarbonyl salt may work either dissolved in the anhydrous alcohol medium, in which case the reaction is conducted in a homogenous phase, or supported on anion exchanging resins as further defined herein-below.

The reaction medium is therefore of heterogeneous type and the reaction proceeds accordings to the corresponding procedures and techniques.

The alcohol solvent medium is chosen from among anhydrous methyl alcohol, ethyl alcohol, isopropyl alcohol, propyl and butyl alcohols.

The alkaline base is chosen from among sodium, potassium and lithium hydroxides, which are admixed to the reaction mixture in the form of solids. Corresponding commercial products may be used for this purpose.

The reaction may be schematically represented by the following equation:

$$1)\quad (II) + CO + 2MOH \xrightarrow[\text{anhydrous alcohol}]{\text{catalyst}} Ar-\underset{\substack{|\\ COOM}}{CH}-CH_3 + MX + H_2O$$

wherein the given symbols are as previously defined and where M represents for K, Na, Li.

The free carboxylic acid (I) is then easily obtained from the alkaline salts by displacement by using strong acids (HCl, $H_2SO_4$), or through extraction with solvents, etc., according to conventional methods.

Effective resins, suitable as carriers for the cobalt hydrocarbonyl salt catalyst, are preferably resins having a styrenic, acrylic or polycondensated matrix. They are characterized by the presence of at least one strongly basic functional group of formula (III) and (IV):

$$-CH_2-N^+(CH_3)_3X^-; \qquad (III)$$

$$-CH_2\underset{\substack{|\\ CH_2CH_2OH}}{N^+}(CH_3)_2X^- \qquad IV)$$

or by a medium basic functional group (V):

$$—CH_2—N(CH_3)_2 \quad (V)$$

wherein X has the meaning stated herein-above.

The resins may, moreover, be either of the gel type or of the porous, isoporous or macroporous type.

The following resins proved to be particularly effective: AMBERLYST A26(III), A27 (III); A29 (IV); AMBERLYTE IRA 402 (III) and IRA 93 (V) (all trade marks of Röhm & Haas); KASTEL A 101 (V) and A 500P (III) (trade marks of MONTEDISON S.p.A.).

The catalysts are cobalt hydrocarbonyl salts of the following formula:

$$Me^{n+}\{Co(CO)_4\}_n \quad (VI)$$

wherein Me represents for a cation of a metal having valence n, such as for instance alkaline metals (Na, K, Li) or cobalt, iron, manganese, etc., that are well known and easily preparable according to conventional methods.

Preferred catalysts are: sodium, cobalt, manganese and iron salts of formula (VI).

Likewise, compounds that are precursors of the above mentioned cobalt hydrocarbonyl salts may also be used.

By the term "precursor" is indicated in this description one or more compounds which, under reaction conditions, will give raise to the above-mentioned cobalt hydrocarbonyl salt.

For instance, the cobalt hydrocarbonyl catalyst salt may be obtained from a cobalt salt such as for instance chloride, sulphate, bromide, etc., an Fe-Mn powder alloy (containing about 80% of Mn) and from sulphurated promoters, in the desired alcohol (methyl, ethyl alcohol, etc.) under a carbon monoxide pressure comprised between 1 and 20 atmospheres and at temperatures comprised between 10°C and 80°C, preferably comprised between 25° and 35°C.

The concentration of cobalt salt in the solution is comprised between 0.3 and 1 mol/litre. For each mole of cobalt salt from 1 to 2 moles of Mn are used in the form of Fe/Mn alloy. This Fe/Mn alloy is ground before hand so that it will pass through a screen having at least 5000 mesh/sq.cm (thus with openings of 0.290 mm).

Preferred sulphurated promoters are sodium sulphite and sodium thiosulphite, which are used in quantities comprised between 0.01 and 0.1 mol/mol of cobalt salt.

The alcohol mixture containing the cobalt salt, the alloy and the sulphurated promoter in the alcohol solvent is kept in a CO atmosphere under vigorous stirring, for sufficient time to allow the complete absorbtion of the CO, this necessitating at least 2 and up to 3 hours. In this way Mn and/or Fe salts of the cobalt hydrocarbonyl are obtained in alcohol solution.

Alternatively, the catalyst alkaline salt of the cobalt hydrocarbonyl may be obtained *"in situ"* from $CO_2(CO)_8$ which, in the basic conditions present in the reaction medium, gives rise to the cobalt hydrocarbonyl salt; or separately the sodium salt may be obtained from $CO_2(CO)_8$ through reduction with a sodium amalgam in an ether solvant (tetrahydrofurane); while the $CO_2(CO)_8$ is prepared for instance from $CoCO_3$ under a CO and hydrogen pressure in petroleum ether.

Supporting the catalyst on the carrier is easily achieved by placing the resin in contact with an alcohol solution of the previously prepared catalyst, optionally after the resin has preliminary been washed with alcohol, preferably with the same alcohol of the reaction, in order to eliminate any moisture contained therein.

This contact is carried out e.g. by immersion of the resin in the catalyst solution.

The resin is used in at least stoichiometric quantity (calculated on the equivalent groups per gram thereof) with the respect to the quantity of catalyst, so that after a short contact time, the catalyst itself will become completely supported by the resin.

The control is provided by the absence of $Co(CO)_4^-$ ions in the I.R. analysis of the solution and by the presence of the same ions in the polymeric matrix. In general, for this purpose, short times of between about 1 and 60 minutes will suffice.

Effective alcohols have proved to be methyl, ethyl and isopropyl alcohol.

Effective alkaline hydroxides have proved to be NaOH and KOH hydroxides.

In order to ensure a selective carbonylation reaction on the secondary carbon atom, the above specified hydroxides are admixed to the reaction mixture in the solid state in the alcohol medium, in quantities equal to at least 2 moles/mole of substrate (II).

The concentration of the alkaline hydroxide is comprised between about 10 and 100 grams per litre of solvent.

Moreover, the addition of the alkaline hydroxides may be carried out in one single operation at the beginning of loading the reactants; in fact no special control of the pH value is necessary. The halide (II) is admixed, as the case may be, either in one single operation or gradually over a period of time, in single batches or continuously.

The concentration of the halide in the alcohol medium is maintained at values comprised between about 40 and 200 g/l of solvent.

The cobalt hydrocarbonyl catalyst salt, whether it is used in solution according to the homogeneous reaction, or supported on a resin as described above according to a heterogeneous reaction, will be added

in a quantity comprised between 1:5 and about 1:500, but preferably comprised between about 1:10 and 1:200, with respect to the halide of formula (II), and calculated as moles of cobalt per moles of halide (II).

The reaction temperature is comprised between about 0°C and 50°C, but is preferably comprised between about 10°C and 35°C.

Moreover, the reaction is completed in a period of between 2 and 24 hours, depending on the parametrical conditions used and on the substrate (II) employed.

The pressure of the carbon monoxide is substantially atmospheric pressure.

The carbonylation reaction is achieved by putting into contact the catalyst, optionally supported on a resin, and an anhydrous alcohol solution containing the Na or K hydroxide and the halide (II) in a carbon monoxide atmosphere. The separation of the reaction product is carried out according to conventional methods.

In the case of operating in a homogeneous phase, i.e., with the cobalt hydrocarbonyl catalyst salt not supported on the resin but dissolved in the alcohol medium, at the end of the reaction, water, acidulated with mineral acids (HC1, $H_2SO_4$), is added and the solution is then extracted by using ethyl ether.

The ether extract is then washed with an aqueous solution saturated with $NaHCO_3$ or with a 10% soda, and the aqueous extract is then acidifed with mineral acids and again extracted by using ethyl ether.

After evaporation of the ether, the desired alpha-aryl-propionic acid is obtained.

In case, however, of operating according to the method of the reaction conducted in an heterogeneous phase, the cobalt hydrocarbonyl catalyst salt, carried by the resin, is separated by filtering and the resin is then washed with a hydroalcohol mixture.

The resin thus separated may be used for a subsequent carbonylation reaction, optionally after restoring the exhausted catalyst. The regeneration of the resin is carried out after a certain number of cycles, when all the supported catalyst is practically exhausted, by simply washing the resin with an aqueous solution of hydrochloric acid.

In this way practically all the cobalt passes into the aqueous solution and the resin, separated by filtering said aqueous acid solution, and washed with water and alcohol, is ready for subsequently supporting the catalyst.

The filtrate combined with the rinsing waters resulting from the rinsing with the hydroalcohol mixture, is then treated as previously described in connection with the reaction in a homogeneous phase.

Halides (II), containing a secondary carbon atom and suitable for use in the carbonylation reaction according to this invention have proved to be: 1-bromo-1-phenylethane; 1-chloro-1-phenylethane; 1-bromo-1-(p-chlorophenyl)-ethane; 1-chloro-1-(6′-methoxy-2′-naphthyl)-ethane, 1-chloro-1-(p-isobutylphenyl)-ethane.

According to a practical processing method, the invention may be carried out as follows:

In a reactor, fitted with a stirrer, a temperature regulating device and a reactant feeding system, the freshly prepared catalyst, optionally supported on a resin, and the alkaline hydroxide, in the desired ratio, are added to the alcohol solvent under a carbon monoxide atmosphere.

The solution of suspension thus obtained is then submitted to stirring. Then, and still under a carbon monoxide atmosphere, halide (II) is added to the reaction mass in the desired ratio.

The reaction mixture is then maintained under stirring at the desired temperature, under CO atmosphere, for the time necessary to achieve absorption of the CO. Once this absorption has stopped, the reaction mixture is treated as previously described in order to separate the products.

The process, of the present invention, proves particularly convenient, as previosly illustrated, for use under mild operational conditions, for the use of inexpensive reactants, for its selectivity and flexibility in application to the secondary halides of the benzyl types, even substituted and its resulting use in a wide range of possible products.

Finally, the catalyst is simple to prepare and, in case of operating with a supported catalyst, the process shows particularly advantageous operational characteristics due to the possibility of operating continuously with easy recovery, regeneration and re-cycling of the catalyst system.

The invention will be further described herein-below by the following examples, given by way of non-limitative illustration.

Example 10 is given in order to evidence the critical nature of the operational parameters of this process. In said example, it will in fact be observed that, when operating in the presence of a hydro-alcohol mixture, instead of an anhydrous alcohol, besides the desired product, substantial quantities of the isomer linear acid (beta-phenyl-propionic acid) will also be obtained.

Example 1

Into a 100 ml flask provided with a magnetic stirrer, a thermometer and a coolant, under a CO atmosphere are introduced respectively: 2 g of NaOH; 22 ml of ethanol; 3 ml of a catalyst solution {2 g of Co as $Co(CO_4)^-$ cobaltate in 100 ml}, prepared from 20 g of $CoCl_2 . 6h_2O$; 0.6 g of $Na_2S.9/10H$ 0; 1.5 g of $Na_2S_2O_3 . 5H_20$; 10 g of an Mn/Fe alloy ground beforehand to pass through a screen of at least 5000 mesh/sg.cm (thus having openings of 0.290 mm); 180 ml of ethanol; and carbon monoxide under atmospheric pressure until the CO absorption is complete.

The temperature was brought to 10°C and 4 g of 1-bromo-1-phenylethane were immediately poured.

The reaction mixture was then subjected to stirring for 16 hours, maintaining during said period the temperature at between 10° and 15°C.

Thereupon water acidulated with HCl was added, and the mixture was then extracted by using ethyl ether. The thus obtained extract was thereupon washed with an aqueous solution of $NaHCO_3$. The resulting alkaline solution was then again acidified and extracted by using ethyl ether. By evaporating the extracting ether 2.5 g of alpha-phenylpropionic acid or hydratropic acid were obtained with a yield equal to 77% with respect to the halide used.

Example 2

Into the same equipment as that described in Example 1, under a CO atmosphere, 2 g of NaoH, 22 ml of ethanol and 3 ml of the catalyst solution prepared as described in Example 1 were introduced.

The temperature was then brought to 30°C and 3.2 g of 1-chloro-1-phenyl-ethane were introduced. The reaction mixture was maintained under stirring for 16 hours while keeping the temperature at 30°C. The reaction mixture was allowed to cool to room temperature and the process was continued as described in Example 1, thereby obtaining 1.85 g of hydratropic acid (yield: 54%).

Example 3

Into the same equipment as that described in Example 1, under a CO atmosphere: 2 g of NaOH, 22 ml of ethanol, and 3 ml of catalyst solution prepared as described in Example 1 were introduced.

The temperature was thereupon brought to 10°C and into the reactor 4.8 g of 1-bromo-1-(p.chlorophenyl)-ethane were introduced. The reaction mixture was maintained under stirring for 16 hours at a temperature comprised between 10°C and 15°C.

Thereupon operating proceded as in Example 1, obtaining 2.8 g of 2-(p.chlorophenyl)-propionic acid having the following formula:

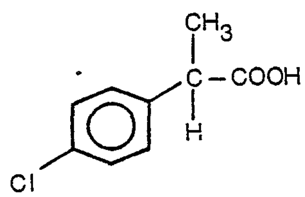

with an actual yield of 69.4%.

Example 4

Into the same equipment as that described in Example 1, under a CO atmosphere,: 1.2 g of NaOH; 50 ml of isopropyl alcohol and 0.25 g of $Co(CO)_8$ were introduced.

The temperature was then brought to 15°C and the reaction mixture was combined with 2.5 g of 1-chloro-1-(6′-methoxy-2′-naphthyl)-ethane of the formula:

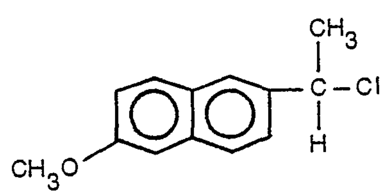

This reaction mixture was maintained under stirring for 8 hours while keeping temperature at 15°C. Thereupon operating proceeded according to that described in Example 1, thereby obtaining 1.5 g of 2-(6′methoxy-2′-naphthyl)-propionic acid of the formula:

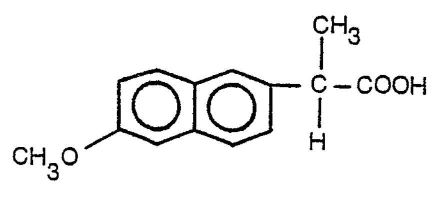

(yield: 57.7%).

Example 5

Into the same apparatus as that described in Example 1, under a CO atmosphere: 2 g of NaOH; 22 ml of ethanol and 3 ml of a catalyst solution prepared as described in Example 1 were introduced. Temperature was brought to 20°C and 3.45 g of 1-chloro-1-(p.isobutylphenyl)-ethane was added having the formula:

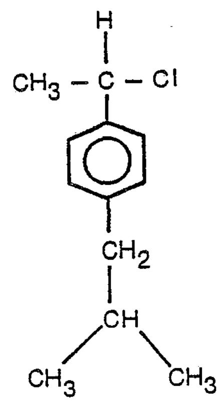

The reaction mixture was thereupon subjected for 16 hours to stirring while keeping the temperature at 20°C. Operating was then proceeded as described in Example 1, using however a 10% aqueous solution of NaOH for extracting the acid fraction. Thereby 2.6 g of 2-(p.isobutylphenyl)-propionic acid were obtained having the formula:

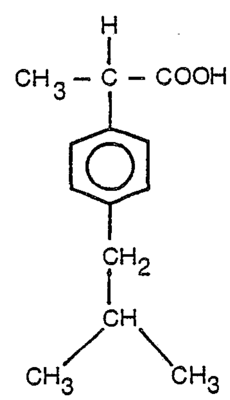

(yield: 71.9%).

Example 6

Into the same apparatus as that described in Example 1, under a CO atmosphere, the following were introduced: 45.5 ml of ethanol; 4.5 ml of a catalyst solution prepared according to the procedures used in example 1, and 4 g of AMBERLIT A26 resin, previously washed with ethanol, and dried.

The suspension was thereupon subjected to stirring for 15 minutes at room temperature so as to completely support the catalyst on the resin (as evidenced by disappearance in the ethanol solution of the I.R. band which is characteristic for cobaltate).

Thereupon 2 g of NaOH were added and, after bringing the temperature to 15°C, 4 g of 1-bromo-1-phenylethane were added. The mixture was then maintained under stirring for 16 hours, with a constant temperature of between 10°C and 15°C.

The resin was then filtered by washing it with a hydroalcohol ethanol/$H_2O$ mixture.

The filtered solution was thereupon treated as described in Example 1, whereby 1.5 g of hydratropic acid (yield: 46.2%) were obtained.

The filtered resin was ready for use in a subsequent cycle.

Example 7

Into the same apparatus as that used in example 1, under a CO atmosphere 2 g of NaOH, 25 ml of ethanol, and: 0.2 g of $NaCo(CO)_4$ were introduced. The mixture was thereupon brought to a temperature of 10°C and admixed with 4 g of 1-bromo-1-phenylethane. The reaction mass was then subjected to stirring

for 16 hours at a temperature maintaining between 10° and 15°C. Operating proceeded as in Example 1, thereby obtaining 2.45 g of hydratropic acid, with an actual yield of 75.5%.

Example 8

Into the same apparatus as that used in Example 1, under a CO atmosphere, 2 g of NaOH, 50 ml of methanol and 0.2 g of $NaCo(CO)_4$ were introduced.

The temperature was thereupon brought to 20°C and into the reactor were introduced 4 grams of 1-bromo-1-phenylethane. The reaction mass was then submitted to stirring for 5 hours at a temperature maintained at 20°C. Operating then proceeded as in Example 1, and 1.72 g of hydratropic acid (yield: 53%) were obtained.

Example 9

Into the same apparatus as that used in Example 1, under a CO atmosphere, the following were introduced: 2 g of NaOH, 22 ml of ethanol and 3 ml of a catalyst solution prepared as described in Example 1. The whole mixture was then brought to a temperature of 23°C and into it 4 g of 1-bromo-1-phenylethane were introduced.

The reaction mass was then subjected to stirring for 5 hours at a temperature maintained at 23°C.

Thereupon, the process was continued as in Example 1, thereby obtaining 2.1 g of hydratropic acid (yield: 64.7%).

Example 10

Into the same apparatus as that described in Example 1, under a CO atmosphere, the following were introduced: 2 g of NaOH, 22 ml of ethanol, 3 ml of a catalyst prepared as in Example 1, and 5 ml of water.

The temperature was thereupon broght to 23°C and the reaction mixture was combined with 4 g of 1-bromo-1-phenylethane. The reaction mixture was then subjected to stirring for 5 hours while keeping the temperature at 23°C.

Thereupon operating proceeded as in Example 1 and 2 g of hydratropic acid (yield: 6.2%) were obtained.

Example 11

Into the same apparatus as that described in Example 1, under a CO atmosphere the following were introduced: 2.5 g of KOH, 22 ml of ethanol and 3 ml of a catalyst prepared as in Example 1. The reaction mixture was thereupon brought to a temperature of 10°C and was then combined with 4 g of 1-bromo-1-phenylethane. The mixture was then submitted to stirring for 16 hours at a temperature comprised between 10°C and 15°C. The process was then continued as described in Example 1, thereby obtaining 1.8 g of hydratropic acid (yield: 56%).

**Claims**

1. A process for the preparation of alkaline salts of alpha-aryl-propionic acids having the formula (I):

$$\begin{array}{l} Ar\text{—}CH\text{—}COOH \\ \quad\quad\ | \\ \quad\quad CH_3 \end{array} \qquad (I)$$

wherein Ar represents an aromatic or heteroaromatic group containing one or several rings linked together, having overall up to 20 carbon atoms, preferably chosen from among phenyl, naphthyl, diphenyl and thienyl groups, in their turn substituted by groups inert under reaction conditions, preferably chosen from among alkyl, cycloalkyl and aryl groups possibly substituted, halogens, alkoxyl groups, phenoxyl groups, ketone groups, by reaction with carbon monoxide of the corresponding secondary halides of formula (II):

$$\begin{array}{l} Ar\text{—}CH\text{—}X \\ \quad\quad\ | \\ \quad\quad CH_3 \end{array}$$

wherein Ar has the meaning defined herein-above and where X is a halogen consisting in Cl or Br, characterized in that the reaction is carried out in an anhydrous alcohol solvent medium consisting of alkanols having up to 4 carbon atoms, in the presence of alkaline hydroxide, and in the presence of a cobalt hydrocarbonyl salt or of one of its precursors, at a temperature comprised between 0°C and 50°C and at a substantially atmosphereic pressure.

2. A process according to claim 1, characterized in that the anhydrous alcohol solvent medium is chosen from the group consisting of: methyl alcohol, ethyl alcohol, isopropyl alcohol, propyl alcohol and butyl alcohol.

3. A process according to claim 1, characterized in that the alkaline hydroxide is chosen from amongst solid sodium, potassium and lithium hydroxides.

4. A process according to claim 1, characterized in that the cobalt hydrocarbonyl salt catalyst has the formula (VI):

$$Me^{n+}\{Co(CO)_4\}_n \qquad (VI)$$

wherein Me represents a metal with valence n, preferably chosen from amongst Na, K, Li, Co, Mn, Fe.

5. A process according to claim 1, characterized in that the reaction is conducted in a homogeneous phase with the cobalt hydrocarbonyl salt catalyst in solution in the anhydrous alcohol solvent medium.

6. A process according to claim 1, characterized in that the reaction is conducted in a heterogeneous phase with the cobalt hydrocarbonyl salt catalyst supported on an anion-exchange resin.

7. A process according to claim 6, characterized in that the cobalt hydrocarbonyl salt catalyst is supported on a resin chosen from amongst those having a styrenic, acrylic and polycondensated matrix, further characterized by the presence of at least one functional group chosen from amongst:

$$-CH_2-N^+(CH_3)_3X-; \qquad (III)$$

$$-CH_2N^+(CH_3)_2X- \qquad (IV)$$
$$|$$
$$CH_2CH_2OH$$

$$-CH_2-N(CH_3)_2 \qquad (V)$$

wherein X has the meaning given herein-above.

8. A process according to claim 7, characterized in that the resin is chosen from amongst the resins therein defined in gelular, porous, isoporous and macroporous form.

9. A process according to claim 6, characterized in that the resin is chosen from amongst: AMBERLYST A26; AMBERLYST A27; AMBERLYST A29; AMBERLITE IRA 401; AMBERLITE IRA 93; KASTEL A101 and KASTEL A 500P.

10. A process according to claim 6, characterised in that the resin is used in at least stoichiometric quantity calculated with reference to the equivalent groups per gram thereof, with respect to the quantity of the cobalt hydrocarbonyl salt catalyst.

11. A process according to claim 6, characterized in that the cobalt hydrocarbonyl salt catalyst is resin-supported by contacting a solution thereof in the alcohol reaction solvent medium with the resin support.

12. A process according to claim 1, characterized in that said process is conducted at a temperature preferably comprised between 10°C and about 35°C.

13. A process according to claim 1, characterized in that the concentration of secondary halide (II) in the alcohol solvent medium is comprised between about 40 and 200 g/l of solvent.

14. A process according to claim 1, characterized in that the alkaline hydroxides are used in a molar ratio of at least 2:1 with respect to the halide (II).

15. A process according to claim 1, characterized in that the concentration of alkaline hydroxide in the anhydrous alcohol solvent medium is comprised between about 10 and 100 g/l of solvent.

16. A process according to claim 1, characterized in that the alkaline hydroxide is introduced into the alcohol reaction solvent medium in the solid state in one single loading operation.

17. A process according to claim 4, characterized in that the cobalt hydrocarbonyl salt catalyst is added in a quantity comprised between about 1:5 and 1:500, but preferably comprised between about 1:10 and 1:200, calculated as moles of cobalt with respect to the moles of halide (II).

18. A process according to claim 4, characterized in that the cobalt hydrocarbonyl salt catalyst is prepared from a cobalt salt, preferably chosen from amongst chloride, bromide and sulphate, from a powder alloy of iron-manganese at 80% of Mn, and from sulphurated promoters, preferably chosen between alkaline sulphites and thiosulphites, in the same solvent medium used for the carbonylation reaction, by reaction with CO at a pressure comprised between 1 and 20 atmospheres and at a temperature comprised between 10°C and 80°C, but preferably comprised between about 25°C and 35°C.

19. A process according to claim 4, characterized in that the cobalt hydrocarbonyl salt catalyst is prepared "in situ" from $Co_2(CO)_8$ in the anhydrous alcohol solvent in the presence of alkaline hydroxides.

20. A process according to claim 1, characterized in that the starting halide of formula (II), as defined in claim 1, is chosen from amongst: 1-bromo-1-phenylethane, 1-chloro-1-phenylethane; 1-bromo-1-(p.chlorophenyl)-ethane; 1-chloro-1-(6'methoxy-2'-naphthyl)-ethane, 1-chloro-1-(p.isobutylphenyl)-ethane.

21. A process for the prepaaration of alpha-aryl-propionic acids or their alkaline salts, having formula (I) as defined in claim 1, according to any one of the claims 1 to 20.

**Patentansprüche**

1. Verfahren zur Herstellung der Alkalisalze von α-Arylpropionsäuren der Formel (I):

$$Ar—\underset{\displaystyle CH_3}{\underset{|}{CH}}—COOH \qquad (I)$$

in der Ar eine aromatische oder heteroaromatische Gruppe ist, welche einen oder mehrere miteinander verbundene Ringe enthält, die insgesamt bis zu 20 Kohlenstoffatome aufweisen, vorzugsweise ausgewählt aus: Phenyl-, Naphthyl-, Diphenyl- und Thienylgruppen, die ihrerseits durch unter den Reaktionsbedingungen inerte Gruppen substituiert sind, vorzugsweise ausgewählt aus: Alkyl-, Cycloalkyl- und Arylgruppen, die gegebenenfalls substituiert sind, Halogene, Alkoxygruppen, Phenoxygruppen, Ketongruppen; durch Reaktion der entsprechenden sekundären Halogenide der Formel (II):

$$Ar—\underset{\displaystyle CH_3}{\underset{|}{CH}}—X$$

in der Ar die obengenannte Bedeutung besitzt und X eines der Halogene, Cl oder Br ist; mit Kohlenmonoxid, dadurch gekennzeichnet, dass die Reaktion in einem wasserfreien Alkohollösungsmittelmedium, bestehend aus Alkanolen mit bis zu 4 Kohlenstoffatomen, in Anwesenheit von Alkalihydroxid und in Anwesenheit eines Kobalthydrocarbonylsalzes oder eines seiner Vorläufer bei einer Temperatur zwischen 0°C und 50°C und bei im wesentlichen atmosphärischem Druck durchgeführt wird.

2. Verfahren nach Anspruch 1, dadurch gekennzeichnet, dass das wasserfreie Alkohollösungsmittelmedium ausgewählt wird aus der Gruppe von: Methylalkohol, Äthylalkohol, Isopropylalkohol, Propylalkohol und Butylalkohol.

3. Verfahren nach Anspruch 1, dadurch gekennzeichnet, dass das Alkalihydroxid ausgewählt wird aus: festem Natrium-, Kalium-bzw. Lithiumhydroxid.

4. Verfahren nach Anspruch 1, dadurch gekennzeichnet, dass der Kobalthydrocarbonylsalz-Katalysator die Formel (VI) besitzt:

$$Me^{n+}\{Co(CO)_4\}_n$$

in der Me ein Metall mit einer Wertigkeit von n ist, vorzugsweise ausgewählt aus: Na, K, Li, Co, Mn, Fe.

5. Verfahren nach Anspruch 1, dadurch gekennzeichnet, dass die Reaktion durchgeführt wird in einer homogenen Phase mit dem Kobalthydrocarbonylsalz-Katalysator in Lösung in dem wasserfreien Alkohollösungsmittelmedium.

6. Verfahren nach Anspruch 1, dadurch gekennzeichnet, dass die Reaktion in einer heterogenen Phase mit dem Kobalthydrocarbonylsalz-Katalysator aufgebracht auf ein Anionenaustauscherharz durchgeführt wird.

7. Verfahren nach Anspruch 6, dadurch gekennzeichnet, dass der Kobalthydrocarbonylsalz-Katalysator aufgebracht ist auf ein Harz, ausgewählt aus Harzen mit einer styrolischen, acrylischen bzw.polykondensierten Matrix, und weiterhin gekennzeichnet durch die Anwesenheit wenigstens einer funktionellen Gruppe, ausgewählt aus:

$$—CH_2—N^+(CH_3)_3X—; \qquad (III)$$

$$—CH_2\underset{\displaystyle CH_2CH_2OH}{\underset{|}{N^+}}(CH_3)_2X—; \qquad (IV)$$

$$—CH_2—N(CH_3)_2 \qquad (V)$$

wobei X die obengenannte Bedeutung besitzt.

8. Verfahren nach Anspruch 7, dadurch gekennzeichnet, dass das Harz ausgewählt wird aus den dort definierten Harzen in gelartiger, poröser, isoporöser und makroporöser Form.

9. Verfahren nach Anspruch 6, dadurch gekennzeichnet, dass das Harz ausgewählt wird aus: AMBERLYST A26; AMBERLYST A27; AMBERLYST A29; AMBERLITE IRA 401; AMBERLITE IRA 93; KASTEL A101; und KASTEL A500P.

10. Verfahren nach Anspruch 6, dadurch gekennzeichnet, dass das Harz in wenigstens stöchiometrischer Menge verwendet wird, berechnet in bezug auf die Äquivalent — Gruppen pro Gramm desselben, bezogen auf die Menge des Kobalthydrocarbonylsalz-Katalysators.

11. Verfahren nach Anspruch 6, dadurch gekennzeichnet, dass der Kobalthydrocarbonylsalz-Katalysator auf den Harzträger aufgebracht wird, indem man eine Lösung desselben in dem Alkoholreaktionslösungsmittelmedium mit dem Harzträger in Berührung bringt.

12. Verfahren nach Anspruch 1, dadurch gekennzeichnet, dass das Verfahren bei einer Temperatur von vorzugsweise zwischen 10°C und etwa 35°C durchgeführt wird.

13. Verfahren nach Anspruch 1, dadurch gekennzeichnet, dass die Konzentration des sekundären Halogenids (II) in dem Alkohollösungsmittelmedium zwischen etwa 40 und 200 g/l Lösungsmittel beträgt.

14. Verfahren nach Anspruch 1, dadurch gekennzeichnet, dass die Alkalihydroxide in einem Molverhältnis von wenigstens 2:1, bezogen auf das Halogenid (II), verwendet werden.

15. Verfahren nach Anspruch 1, dadurch gekennzeichnet, dass die Konzentration des Alkalihydroxids in dem wasserfreien Alkohollösungsmittelmedium zwischen etwa 10 und 100 g/l Lösungsmittel beträgt.

16. Verfahren nach Anspruch 1, dadurch gekennzeichnet, dass das Alkalihydroxid in fester Form und in einem einzigen Beschickungsschritt in das Alkoholreaktionslösungsmittelmedium gegeben wird.

17. Verfahren nach Anspruch 4, dadurch gekennzeichnet, dass der Kobalthydrocarbonylsalz-Katalysator in einer Menge zwischen etwa 1:5 bis 1:500, jedoch vorzugsweise zwischen etwa 1:10 bis 1:200, berechnet als Mole Kobalt bezogen auf die Mole Halogenid (II), zugegeben wird.

18. Verfahren nach Anspruch 4, dadurch gekennzeichnet, dass der Kobalthydrocarbonylsalz-Katalysator hergestellt wird aus einem Kobaltsalz, vorzugsweise ausgewählt aus: Chlorid, Bromid und Sulfat, aus einer pulverförmigen Eisen-Mangan-Legierung mit 80% Mn, und aus Schwefelpromotoren, vorzugsweise ausgewählt aus: Alkalisulfiten und-Thiosulfiten, im gleichen Lösungsmittelmedium, das bei der Carbonylierungsreaktion verwendet wird, durch Reaktion mit CO bei einem Druck zwichen 1 und 20 Atmosphären und einer Temperatur zwischen etwa 10°C und 80°C, vorzugsweise jedoch zwischen etwa 25°C und 35°C.

19. Verfahren nach Anspruch 4, dadurch gekennzeichnet, dass der Kobalthydrocarbonylsalz-Katalysator "in situ" hergestellt wird aus $CO_2(CO)_8$ in dem wasserfreien Alkohollösungsmittel in Anwesenheit von Alkalihydroxiden.

20. Verfahren nach Anspruch 1, dadurch gekennzeichnet, dass das Ausgangs-Halogenid gemäss der in Anspruch 1 definierten Formel (II) ausgewählt wird aus: 1-Brom-1-phenyläthan, 1-Chlor-1-phenyläthan, 1-Brom-1-(p-chlorphenyl)-äthan, 1-Chlor-1-(6'-methoxy-2'-naphthyl)-äthan, 1-Chlor-1-(p-isobutylphenyl)-äthan.

21. Verfahren zur Herstellung von α-Arylpropionsäuren oder deren Alkalisalzen, dargestellt durch die in Anspruch 1 definierte Formel (I), nach einem der vorhergehenden Ansprüche 1 bis 20.

**Revendications**

1. Un procédé de préparation de sels alcalins d'acides alpha-aryl-propioniques ayant la formule (I):

$$\begin{array}{c} Ar—CH—COOH \\ | \\ CH_3 \end{array} \qquad (I)$$

dans laquelle:

Ar représente un groupe aromatique ou hétéro-aromatique contenant un ou plusieurs cycles liés entre eux, possédant en tout jusqu'à 20 atomes de carbone, choisis de préférence parmi les groupes phényl, naphtyle, diphényle et thiényle, substitués à leur tour par des groupes inertes dans les conditions de la réaction, choisis de préférence parmi les groupes alkyle, cycloalkyle et aryle, éventuellement substitués, des halogènes, des groupes alkoxyles, des groupes phénoxyles, des groupes cétones, par réaction, avec le monoxyde de carbone, des halogénures secondaires correspondants de formule (II):

$$\begin{array}{c} Ar—CH—X \\ | \\ CH_3 \end{array} \qquad (II)$$

dans laquelle:

Ar a la signification définie ci-dessus, et

X représente un halogène consistant en Cl ou Br, caractérisé en ce que la réaction est mise en ouevre dans un milieu solvant alcoolique anhydre constitué d'alcanols possédant jusqu'à 4 atomes de carbone, en présence d'hydroxyde alcalin et en présence d'un sel de colbalt-hydrocarbonyle ou de l'un de ses précurseurs, à une température comprise entre 0°C et 50°C et à une pression sensiblement atmosphérique.

2. Un procédé selon la revendication 1, caractérisé en ce que le milieu solvant alcool anhydre est choisi dans le groupe constitué par alcool méthylique, alcool éthylique, alcool isopropylique, alcool propylique et alcool butylique.

3. Un procédé selon la revendication 1, caractérisé en ce que l'hydroxyde alcalin est choisi parmi les hydroxydes de sodium, de potassium et de lithium solides.

4. Un procédé selon la revendication 1, caractérisé en ce que le catalyseur à base de sel de cobalt-hydrocarbonyle a la formule (VI):

$$Me^{n+}\{Co(CO)_4\}_n \qquad (VI)$$

dans laquelle:

Me représente un métal ayant une valence n, choisi de préférence parmi Na, K, Li, Co, Mn, Fe.

5. Un procédé selon la revendication 1, caractérisé en ce que la réaction est conduite en phase homogène, le catalyseur à base de sel de cobalt-hydrocarbonyle étant en solution dans le milieu solvant alcoolique anhydre.

6. Un procédé selon la revendication 1, caractérisé en ce que la réaction est conduite en phase hétérogène, le catalyseur à base de sel de cobalt-hydrocarbonyle étant fixé sur un support consistant en une résine échangeuse d'anion.

7. Un procédé selon la revendication 6, caractérisé en ce que le catalyseur à base de cobalt-hydrocarbonyle est fixé sur un support formé par un une résine choisie parmi celles ayant une matrice styrénique, acrylique ou polycondensée, caractérisé en outre par la présence d'au moins un groupe fonctionnel choisi parmi:

$$—CH_2—N^+(CH_3)_3X—; \qquad (III)$$

$$—CH_2N^+(CH_3)_2X—; \qquad (IV)$$
$$|$$
$$CH_2CH_2OH$$

$$—CH_2—N(CH_3)_2 \qquad (V)$$

dans lesquelles X a la signification donnée ci-dessus.

8. Un procédé selon la revendication 7, caractérisé en ce que la résine est choisie parmi les résines définies sous une forme gélatineuse, poreuse, isoporeuse et macroporeuse.

9. Un procédé selon la revendication 6, caractérisé en ce que la résine est choisie parmi AMBERLYST A26; AMBERLYST A27; AMBERLYST A29; AMBERLITE IRA 401; AMBERLITE IRA 93; KASTEL A101 et KASTEL A 500P.

10. Un procédé selon la revendication 6, caractérisé en ce que la résine est utilisée au moins en quantité stoechiométrique calculée par rapport aux groupes équivalents par gramme de ceux-ci et par rapport à la quantité de catalyseur à base de sel de cobalt-hydrocarbonyle.

11. Un procédé selon la revendication 6, caractérisé en ce que le catalyseur formé de sel de cobalt-hydrocarbonyle est fixé sur un support formé par un résine par mise en contact d'une solution de celle-ci dans le milieu solvant réactionnel alcoolique avec le support de résine.

12. Un procédé selon la revendication 8, caractérisé en ce que procédé conduit à une température comprise de préférence entre 10°C et environ 35°C.

13. Un procédé selon la revendication 1, caractérisé en ce que la concentration de l'halogénure secondaire (II) dans le milieu solvant aqueux est comprise entre 40 et 200 g/l de solvant.

14. Un procédé selon la revendication 1, caractérisé en ce que les hydroxydes alcalins sont utilisés dans un rapport molaire d'au moins 2/1 par rapport à l'halogénure (II).

15. Un procédé selon la revendication 1, caractérisé en ce que la concentration de l'hydroxyde alcalin dans le mileiu solvant alcoolique anhydre est comprise entre environ 10 et 100 g/l de solvant.

16. Un procédé selon la revendication 1, caractérisé en ce que l'hydroxyde alcalin est introduit dans le milieu solvant réactionnel alcoolique à l'état solide en une seule opération de chargement.

17. Un procédé selon la revendication 4, caractérisé en ce que le catalyseur de sel de cobalt-hydrocarbonyle est ajouté en quantité comprise entre environ 1/5 et 1/500, mais de préférence comprise entre environ 1/10 et 1/200, calculée en moles de cobalt par rapport aus moles de l'halogénure (II).

18. Un procédé selon la revendication 4, caractérisé en ce que le catalyseur formé de sel de cobalt-hydrcarbonyle est préparé à partir d'un sel de cobalt, de préférence choisi parmi chlorure, bromure et sulfate, à partir d'une poudre d'alliage fer-manganèse à 80% de Mn et à partir de promoteurs sulfurés de préférence choisi entre les sulfites et les thiosulfites alcalins, dans le même milieu solvant que celui utilisé pour la réaction de carbonylation par réaction avec CO sous une pression comprise entre 1 et 20 atmosphères et à une température comprise entre environ 10°C et 80°C, et comprise de préférence entre environ 25°C et 35°C.

19. Un procédé selon la revendication 4, caractérisé en ce que le catalyseur de sel de cobalt-hydrocarbonyle est préparé "in situ" à partir de $Co_2(CO)_8$ dans le solvant alcool anhydre en présence d'hydroxy-des alcalins.

20. Un procédé selon la revendication 1, caractérisé en ce que l'halogénure de départ de formule (II) tel que défini dans la revendication 1, est choisi parmi 1-bromo-1-phényléthane, 1-chloro-1-phényléthane, 1-bromo-1-(p-chlorophényl)éthane, 1-chloro-1-(6'-méthoxy-2'-naphtyl)éthane, 1-chloro-1-(p-isobutylphényl)éthane.

21. Un procédé pour la préparation d'acides alpha-aryl-propioniques ou de leurs sels alcalins ayant la formule (I) telle que définie dans la revendication 1, selon une quelconque des revendications 1 à 20.